# EUROPEAN PATENT SPECIFICATION

(11) **EP 1 983 968 B1**
(45) Date of publication and mention of the grant of the patent: **07.11.2012**
(21) Application number: 06802935.4
(22) Date of filing: 06.09.2006
(51) Int. Cl.: A61K 9/48

(54) **GELATIN CAPSULES CONTAINING ACTIVES**
WIRKSTOFFE ENTHALTENDE GELATINEKAPSELN
CAPSULES DE GELATINE CONTENANT DES SUBSTANCES ACTIVES

(30) Priority: 08.09.2005 US 222304
(43) Date of publication of application: 29.10.2008
(73) Proprietor: Kraft Foods Global Brands LLC, Northfield, IL 60093 (US)
(72) Inventor: SAKANISHI, Hideki, Saitama 336-0025 (JP); MISTRY, Atul, Branchburg, NJ 08876 (US)
(74) Representative: Wilson Gunn
(86) International application number: PCT/US2006/034502
(87) International publication number: WO 2007/030431

(56) References cited:
- EP-A1- 0 039 879
- EP-A1- 0 116 743
- FR-A- 2 447 720
- US-A- 3 071 513
- US-A- 4 940 499
- US-A- 5 188 688

## Description

### BACKGROUND OF THF INVENTION

### Field of the Invention

The present invention relates to the surface treatment of gelatin capsules whereby the shelf life is enhanced as well as the overall organoleptic perception of the actives contained within the capsule.

### Background of Related Technology

Gelatin capsules exist in both hard and soft forms. They are typically used to deliver solid or liquid components such as vitamins, drugs and breath freshening materials. Gelatin capsules suffer from the drawback, however, of being very sensitive to moisture or other solvents which result in the capsule surface becoming sticky and commercially unacceptable. In some instances, the stability of the capsule can be affected such that long term storage of the active contained within is negatively affected, which in turn can compromise the activity of the active. Any attempts to alter the surface to make the capsule less acceptable to moisture must be careful not to delay the dissolution rate, and thus delay the release of the material within, to a speed which is commercially unacceptable.

Additionally, current commercially available capsules contain the actives within the open volume formed by the capsule shell. Thus, in order to release the active and experience its intended affects, it is necessary to dissolve the capsule or otherwise disrupt the capsule shell once placed in the mouth or swallowed. There is a need for gelatin capsules which provide enhanced stability of the capsule shell, and therefore enhancing the protection of the actives within, without deleteriously affecting the dissolution properties of the gelatin capsule.

### SUMMARY OF THE INVENTION

Surface treatment of the gelatin capsules is accomplished using a number of different materials and methodologies. Additionally, surface treatment of the gelatin capsules with compositions which include taste modifiers such as sweeteners and/or flavors, are also included in order to enhance the perception of the capsule when ingested.

In one embodiment the invention relates to an ingestible capsule which includes a denatured gelatin shell and an active contained within the shell. The gelatin's shell has been subjected to a low volatile, food grade solvent which denatures the gelatin, thereby increasing its resistance to thermal reversibility and provides a harder, more structurally sound capsule shell. Coating compositions containing the low volatile food grade solvent may also contain other additives such as sweeteners, flavors and breath freshening components which serve to impart their properties for enhanced organoleptic perception by the user.

Other embodiments include within the surface coating composition components which are hydrophobic in nature such as waxes, polymers, shellac and combinations of these materials.

In another embodiment of the invention the surface coating of the capsule includes a hydrophobic material such as those referred to above and optionally other components such as sweeteners, flavors and breath freshening components.

### DETAILED DESCRIPTION OF THE INVENTION

As used herein, the transitional term "comprising," (and "comprises," etc.) which is synonymous with "including," "containing," and "characterized by," is inclusive or openended, and does not exclude additionally, unrecited elements or method steps, regardless of its use in the preamble or the body of the claim.

A variety of gelatin compositions may be employed in the present invention. For example, type A gelatin and type B gelatin or combinations of these types are among those that maybe employed. Type A gelatin is generally understood to be made from pigskin via an acidic process. Type B gelatin is generally understood to be made from cattle hide and bones, via a basic process.

The gelatin may be present in amounts of about 10 to about 70% weight of the capsule shell. Advantageously, the amount of gelatin present may be about 20 to about 60% and even more advantageously about 20 to about 40% by weight of the capsule shell. In embodiments which include seamless capsules, it may be desirable to include gelatin in amounts of greater than about 60% by weight of the shell, as disclosed in co-pending U.S. 2005 0,144,331.

Suitable compositions for treatment of the gelatin capsule shells include alcohol such as ethanol, propanol, decanol, dodecanol, heptanol, hexanol and combinations thereof. These solvent materials can be combined with one or more components such as flavors, sweeteners, breath freshening components, plasticizers, viscosity modifiers, surfactants and coloring agents to modify the taste, texture or other organoleptic, sensory perceptions of the consumer.

It has been discovered that the use of materials which denature the gelatin, such as alcohol compositions, result in a harder gelatin surface which also has great resistance to thermo-reversibility. The alcohol compositions may also include one or more of the aforementioned components such that the capsule becomes coated with the component. In the case of taste modifier components, such as flavors, sweeteners and breath-freshening components, their presence on the surface of the capsule serve to provide an immediate taste impact when placed in the mouth. In general, the alcohol *per se* gradually volatilizes, leaving any components which it carried on the surface of the capsule.

The alcohol content of the surface treatment compositions may be about 0.05% to about 3.0% by weight of the surface treatment composition. The amount of taste modifies present in the surface treatment composition may also vary depending on the chosen component. When present in the surface treatment composition, the amount of flavor may be about 0.02% to about 5.0%, and desirably about 0.8% to about 1.8% by weight of the surface treatment composition. The amount of sweetener present may be about 0.01 % to about 3.0%, and desirably about 0.1% to about 0.6% by weight of the surface treatment composition. The amount of breath freshening components present may be about 0.001% to about 5%, and desirably about 0.1% to about 2.0% by weight of the surface treatment compositions. As mentioned, combinations of these and other taste modifiers may be present in the surface treatment composition.

In addition to being present in the surface treatment compositions, which are applied to the surface of the capsule, the aforementioned taste modifiers, including flavors, sweeteners, breath-freshening components and combinations thereof, may also be incorporated within the cavity of the capsule.

Other surface treatment compositions which are protective in nature include hydrophobic materials such as waxes, polymers and shellac. Useful waxes include beeswax, candelilla wax, carnuba wax and combinations thereof. Combinations of waxes, polymers and shellac are also useful. In some embodiments, the waxes employed are low melting waxes, such as those which melt below 60°C and preferably those which melt between about 45°C to about 55°C. The amount of wax present may vary from 0% to about 25%, and advantageously may be present in amounts from about 7% to about 9.5% by weight of the surface treatment composition.

Higher melting point waxes may also be used, in concentrations up to about 5% by weight of the surface treatment composition. These waxes include beeswax, vegetable wax, candelilla wax, carnuba wax, petroleum waxes such as paraffin, and mixtures thereof.

The surface treatment compositions may also include polymers. Examples of such polymers are cellulose acetate, polyamide, polyethylene, polyethylene terephthalate, polypropylenem polyurethane, polyvinyl acetate, polyvinyl chloride, silicone rubber, latex, polyhydroxybutyrate, polyhydroxyvalerate, teflon, polylactic acid or polyglycolic acid and copolymers thereof, copolymers such as ethylene vinyl acetate (EVA), styrene-butadienestyrene (SBS) and styrene-isoprene-styrene (SIS). The amount of polymer present in the surface treatment composition may be about 0.1% to about 40%, and desirably about 2% to about 10% by weight of the total composition.

The flavoring agents which may be used include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof. Nonlimiting representative flavor oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, and fruit essences including apple, pear, peach, grape, strawberry, raspberry, cherry, plum, pineapple, apricot and so forth. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with the cooling agents, described herein below.

Other useful flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used. This publication is incorporated herein by reference. This may include natural as well as synthetic flavors.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin), cherry, grape, strawberry shortcake, and mixtures thereof.

In some embodiments, the flavoring agent may be employed in either liquid form and/or dried form. When employed in the latter form, suitable drying means such as spray drying the oil may be used. Alternatively, the flavoring agent may be absorbed onto water soluble materials, such as cellulose, starch, sugar, maltodextrin, gum arabic and so forth or may be encapsulated. The actual techniques for preparing such dried forms are well-known.

In some embodiments, the flavoring agents may be used in many distinct physical forms well-known in the art to provide an initial burst of flavor and/or a prolonged sensation of flavor. Without being limited thereto, such physical forms include free forms, such as spray dried, powdered, beaded forms, encapsulated forms, and mixtures thereof.

The amount of flavoring agent employed herein may be a matter of preference subject to such factors as the type of final composition, the individual flavor, and the strength of flavor desired. Thus, the amount of flavoring may be varied in order to obtain the result desired in the final product and such variations are within the capabilities of those skilled in the art without the need for undue experimentation. The flavoring agent is generally present in amounts from about 0.02% to about 5%, and more specifically from about 0.1% to about 2%, and even more specifically, from about 0.8% to about 1.8%, by weight of the total composition.

The sweetening agents used may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:
(a) water-soluble sweetening agents such as dihydrochalcones, monellin, steviosides, glycyrrhizin, dihydroflavenol, and sugar alcohols such as sorbitol, mannitol, maltitol, and L-aminodicarboxylic acid aminoalkenoic acid ester amides, such as those disclosed in U.S. Pat. No. 4,619,834, which disclosure is incorporated herein by reference, and mixtures thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
(c) dipeptide based sweeteners, such as L-aspartic acid derived sweeteners, such as L-aspartyl-L-phenylalanine methyl ester (Aspartame) and materials described in U.S. Pat. No. 3,492,131, L-alphaaspartyl-N-(2,2,4,4-tetramethyl-3-thietanyl)-D-alaninamide hydrate (Alitame), N-[N-(3,3-dimethylbutyl)-L-aspartyl]-L-phenylalanine 1-methyl ester (Neotame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(l-cyclohexen)-alanine, and mixtures thereof;
(d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-f uranoside, or 4,1'-dichloro-4,1'-dideoxygalactosucrose; 1',6'-dichlorol',6'-dideoxysucrose; 4-chloro-4-deoxy-alpha-D-galactopyranosyl-1,6-dichloro-1,6-dideoxy-beta-D- fructofuranoside, or 4,l',6'-trichloro-4,1',6'-trideoxygalactosucrose; 4,6-dichloro-4,6-dideoxy-alpha-D-galactopyranosyl-6-chloro-6-deoxy-beta-D- fructofuranoside, or 4,6,6'-trichloro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideox y-beta-D-fructofuranoside, or 4,6,1',6'-tetrachloro4,6,1',6'-tetradeoxygalacto-sucrose; and 4,6,1',6'-tetradeoxy-sucrose, and mixtures thereof;
(e) protein based sweeteners such as thaumaoccous danielli (Thaumatin I and II); and
(f) the naturally occurring sweetener monatin (2-hydroxy-2-(indol-3-ylmethyl)-4-aminoglutaric acid) and its derivatives.

Sensates also may be included, such as cooling, warming and/or tingling agents. With respect to cooling agents, a variety of well known cooling agents may be employed. For example, among the useful cooling agents are included menthol, xylitol, erythritol, menthane, menthone, ketals, menthone ketals, menthone glycerol ketals, substituted p-menthanes, acyclic carboxamides, substituted cyclohexanamides, substituted cyclohaxane carboxamides, substituted ureas and sulfonamides, substituted menthanols, hydroxymethyl and hydroxymethyl derivatives of p-menthane, 2-mercapto-cyclo-decanone, 2-isoprpanyl-5-methylcyclohexanol, hydroxycarboxylic acids with 2-6 carbon atoms, cyclohexanamides, menthyl acetate, menthyl lactate, methyl salicylate, N,2,3-trimethyl-2-isopropyl butanamide (WS-23), N-ethyl-p-menthane-3-carboxamide (WS-3), menthyl succinate, 3,1-menthoxypropane 1,2-diol, glutarate esters, among others, and combinations thereof. These and other suitable cooling agents are further described in the following U.S. patents: U.S. 4,230,688 and 4,032,661 to Rowsell et al.; 4,459,425 to Amano et al.; 4,136,163 to Watson et al.; and 5,266,592 to Grub et al. Cooling agents are generally present in amount of 0.01% to about 10.0%.

Warming agents may be selected from a wide variety of compounds known to provide the sensory signal of warming to the individual user. These compounds offer the perceived sensation of warmth, particularly in the oral cavity, and often enhance the perception of flavors, sweeteners and other organoleptic components. Useful warming agents include those having at least one allyl vinyl component, which may bind to oral receptors. Examples of suitable warming agents include, but are not limited to: vanillyl alcohol n-butylether (TK-1000, supplied by Takasago Perfumery Company Ltd., Tokyo, Japan); vanillyl alcohol n-propylether; vanillyl alcohol isopropylether; vanillyl alcohol isobutylether; vanillyl alcohol n-aminoether; vanillyl alcohol isoamylether; vanillyl alcohol n-hexylether; vanillyl alcohol methylether; vanillyl alcohol ethylether; gingerol; shogaol; paradol; zingerone; capsaicin; dihydrocapsaicin; nordihydrocapsaicin; homocapsaicin; homodihydrocapsaicin; ethanol; isopropyl alcohol; iso-amylalcohol; benzyl alcohol; glycerine; chloroform; eugenol; cinnamon oil; cinnamic aldehyde; phosphate derivatives thereof; and combinations thereof.

Tingling agents may provide a tingling, stinging or numbing sensation to the user. Tingling agents include, but are not limited to: Jambu Oleoresin or para cress (Spilanthes sp.), in which the active ingredient is Spilanthol; Japanese pepper extract (Zanthoxylum peperitum), including the ingredients known as Saanshool-I, Saanshool-II and Sanshoamide; black pepper extract (piper nigrum), including the active ingredients chavicine and piperine; Echinacea extract; Northern Prickly Ash extract; and red pepper oleoresin. Tingling agents are described in U.S. Patent No. 6,780,443 to Nakatsu et al., U.S. Patent No. 5,407,665 to McLaughlin et al., U.S. Patent No. 6,159,509 to Johnson et al. and U.S. Patent No. 5,545,424 to Nakatsu et al.,

A variety of nutritional supplements may also be included in the compositions of the invention. Virtually any vitamin or mineral may be included. For example, vitamin A, vitamin C, vitamin D, vitamin E, vitamin K, vitamin B₆, vitamin B₁₂, thiamine, riboflavin, biotin, folic acid, niacin, pantothenic acid, sodium, potassium, calcium, magnesium, phosphorus, sulfur, chlorine, iron, copper, iodine, zinc, selenium, manganese, chromium, molybdenum, fluorine, cobalt and combinations thereof, may be used.

Coloring agents may be used in amounts effective to produce a desired color. The coloring agents may include pigments which may be incorporated in amounts up to about 6%, by weight of the composition. For example, titanium dioxide may be incorporated in amounts up to about 2%, and preferably less than about 1%, by weight of the composition. The colorants may also include natural food colors and dyes suitable for food, drug and cosmetic applications. These colorants are known as F.D.& C. dyes and lakes. The materials acceptable for the foregoing uses are preferably water-soluble. Illustrative nonlimiting examples include the indigoid dye known as F.D.& C. Blue No.2, which is the disodium salt of 5,5-indigotindisulfonic acid. Similarly, the dye known as F.D.& C. Green No.1 comprises a triphenylmethane dye and is the monosodium salt of 4-[4-(N-ethyl-p-sulfoniumbenzylamino) diphenylinethylene]-[1-(N-ethyl -N-p-sulfoniumbenzyl)-delta-2,5-cyclohexadieneimine]. A full recitation of all F.D.& C. colorants and their corresponding chemical structures may be found in the Kirk-Othmer Encyclopedia of Chemical Technology, 3rd Edition, in volume 5 at pages 857-884, which text is incorporated herein by reference.

Examples of useful drugs (medicaments) include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, antihistamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, antineoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra®, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocryptine or nicotine, sympatholytics, tremor preparations, urinary tract agents, vasodilators, laxatives, antacids, ion exchange resins, antipyretics, appetite suppressants, expectorants, anti-anxiety agents, anti-ulcer agents, anti-inflammatory substances, coronary dilators, cerebral dilators, peripheral vasodilators, psychotropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyperand hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

### EXAMPLE 1

This example provides a gelatin capsule which is surface treated (coated) with a surface treatment composition of the invention which includes an alcohol composition, flavor and sweetener.

A mixture of 98.8 w/w% of ethanol, 1.0 w/w% of peppermint oil and 0.2w/w% of aspartame is mixed together. Capsules are put in a coating-pan with 500 grams of the mixture and rotated at about 25 rpm. About 1% of the mixture is also poured on the surface of the capsules. The coated capsules are air dried. This cycle is repeated five times. The hardness of coated capsules is compared to the same capsules but which are non-coated. Measurements are performed with Texture Analyzer TA-XT2 (Stable Micro Systems Company). The gelatin capsule is denaturization and the coating is harder. The capsule is more resistant to resistant to thermo-reversibility.

### EXAMPLE 2

Another composition in accordance with the invention is prepared which includes 98.8 w/w% of menthol, 0.5w/w% of natural lemon oil and 0.2w/w% of aspartame. This composition is mixed well and used to coat gelatin capsules in the same manner as Example 1. The coated capsule is harder subsequent to the application of the coating.

## Claims

1. A capsule for release of a breath-freshening component in the mouth comprising:
a gelatine shell housing a cavity of said gelatine capsule, said gelatine shell formulated for dissolution in the mouth and having a surface structure hardened by denaturization of said gelatine shell and increased resistance to therm-reversibility due to said denaturisation, said cavity containing a breath-freshening component; and
a coating applied to said gelatine shell comprising a surface treatment composition, where said surface treatment composition comprises a denaturing alcohol selected from ethanol, propanol, decanol, dodecanol, heptanol, hexanol and combinations thereof and at least one active selected from flavors, sweeteners, breath freshening components, cooling agents, warming agents, tingling agents and combinations thereof;
wherein said surface treatment provides immediate flavor impact in the mouth and enhances the organoleptic perception of said breath-freshening component contained within said cavity of said capsule as it is released in the mouth.

2. The capsule of claim 1, wherein the capsule comprises gelatine in an amount of about 30% to about 60% by weight of the shell.

3. The capsule of claim 1, wherein the shell is dry to the touch.

4. The capsule of either of claims 1 or 3, wherein the gelatine shell is rendered harder due to the solvent composition, as compared to the gelatin shell in the absence of exposure to the solvent composition.

5. The capsule of any of claims 1, 3 or 4, further comprising one or more flavors.

6. The capsule of claim 1, wherein a food grade solvent composition is coating the shell, and of any of claims 3-5, wherein a material selected from flavors, sweeteners, cooling compounds, plasticizers, humectants, viscosity modifiers, solvents and combinations thereof is present in one or more of the shell, the coating or contained within the capsule.

## Patentansprüche

1. Kapsel zur Freisetzung einer atemerfrischenden Komponente im Mund, umfassend:
eine Gelatinehülle, die einen Hohlraum der Gelatinekapsel umschließt, wobei die Gelatinehülle zur Auflösung im Mund formuliert ist und eine durch Denaturierung der Gelatinehülle gehärtete Oberflächenstruktur und eine erhöhte Widerstandsfähigkeit gegen Thermoreversibilität aufgrund dieser Denaturierung aufweist, wobei der Hohlraum eine atemerfrischende Komponente enthält; und
eine Beschichtung, aufgebracht auf die Gelatinehülle, umfassend eine Zusammensetzung zur Oberflächenbehandlung, wobei die Zusammensetzung zur Oberflächenbehandlung einen denaturierenden Alkohol, ausgewählt aus Ethanol, Propanol, Decanol, Dodecanol, Heptanol, Hexanol und Kombinationen davon, und mindestens einen Wirkstoff, ausgewählt aus Aromastoffen, Süßungsmitteln, atemerfrischenden Komponenten, kühlenden Mitteln, wärmenden Mitteln, prickelnden Mitteln und Kombinationen davon, umfasst;
wobei die Oberflächenbehandlung eine sofortige Auswirkung auf den Geschmack im Mund hat und die organoleptische Wahrnehmung der atemerfrischenden Komponente, die in dem Hohlraum der Kapsel enthalten ist, verbessert, während sie im Mund freigesetzt wird.

2. Kapsel nach Anspruch 1, wobei die Kapsel Gelatine in einer Menge von etwa 30 Gew.-% bis etwa 60 Gew.-% bezogen auf die Hülle enthält.

3. Kapsel nach Anspruch 1, wobei sich die Hülle trocken anfühlt.

4. Kapsel nach einem der Ansprüche 1 oder 3, wobei die Gelatinehülle infolge der Lösungsmittelzusammensetzung härter gemacht wird, im Vergleich zur Gelatinehülle ohne Einwirkung der Lösungsmittelzusammensetzung.

5. Kapsel nach einem der Ansprüche 1, 3 oder 4, weiterhin umfassend einen oder mehrere Aromastoffe.

6. Kapsel nach Anspruch 1, wobei eine Lösungsmittelzusammensetzung in Lebensmittelqualität die Hülle beschichtet, und nach einem der Ansprüche 3 bis 5, wobei ein Stoff, ausgewählt aus Aromastoffen, Süßungsmitteln, kühlenden Verbindungen, Weichmachern, Feuchthaltemitteln, Viskositätsmodifizierern, Lösungsmitteln und Kombinationen davon, in mindestens einem von Hülle oder Beschichtung vorliegt oder in der Kapsel enthalten ist.

## Revendications

1. Capsule destinée à libérer un composant de rafraîchissement d'haleine dans la bouche comprenant :
une coque en gélatine logeant une cavité de ladite capsule en gélatine, ladite coque en gélatine étant formulée pour une dissolution dans la bouche et ayant une structure de surface durcie par dénaturation de ladite coque en gélatine et une résistance plus élevée à une réversibilité thermique due à ladite dénaturation, ladite cavité contenant un composant de rafraîchissement d'haleine ; et
un revêtement appliqué à ladite coque en gélatine comprenant une composition de traitement de surface, ladite composition de traitement de surface comprenant un alcool dénaturant sélectionné parmi l'éthanol, le propanol, le décanol, le dodécanol, l'heptanol, l'hexanol, et des combinaisons de ceux-ci, et au moins un actif sélectionné parmi des arômes, des édulcorants, des composants de rafraîchissement d'haleine, des agents de refroidissement, des agents chauffants, des agents piquants et des combinaisons de ceux-ci ;
dans laquelle ledit traitement de surface a un impact aromatisant immédiat dans la bouche et améliore la perception organoleptique dudit composant de rafraîchissement d'haleine contenu dans ladite cavité de ladite capsule alors qu'il est libéré dans la bouche.

2. Capsule selon la revendication 1, laquelle capsule comprend de la gélatine en une quantité d'environ 30 % à environ 60 % en poids de la coque.

3. Capsule selon la revendication 1, dans laquelle la coque est sèche au toucher.

4. Capsule selon l'une ou l'autre des revendications 1 et 3, dans laquelle la coque en gélatine est rendue plus dure du fait de la composition de solvant, comparée à la coque en gélatine en l'absence d'une exposition à la composition de solvant.

5. Capsule selon l'une quelconque des revendications 1, 3 ou 4, comprenant en outre un ou plusieurs arômes.

6. Capsule selon la revendication 1, dans laquelle une composition de solvant de qualité alimentaire recouvre la coque, et selon l'une quelconque des revendications 3 à 5, dans laquelle un matériau sélectionné parmi des arômes, des édulcorants, des composés de refroidissement, des plastifiants, des humectants, des modificateurs de viscosité, des solvants et des combinaisons de ceux-ci est présent dans un ou plusieurs de la coque, du revêtement ou est contenu dans la capsule.
